# EUROPEAN PATENT APPLICATION

(11) **EP 1 537 831 A1**
(43) Date of publication of application: **08.06.2005**
(21) Application number: 03447280.3
(22) Date of filing: 04.12.2003
(51) Int. Cl.: A61B 19/00, A61B 6/04, A61B 5/055

(54) **Head immobilisation assembly for patient positioning in radiation therapy**

(71) Applicant: Orfit Industries, 2110 Wijnegem (BE)
(72) Inventor: Cuypers, Steven, 2110 Wijnegem (BE); Bogdanov, Bogdan, 2110 Wijnegem (BE)
(74) Representative: Luys, Marie-José

(57) **Abstract**

This invention relates to a patient immobilisation assembly for immobilising at least part of a patient, the immobilisation assembly comprising a mask (1) made of a thermoplastic material, a fixation plate (3, 12, 13) and connecting means (10, 11, 14, 15, 16) for connecting the mask (1) to the fixation plate (3, 12, 13) in view of fixing the position of the mask with respect to the fixation plate, the mask being provided to cover at least partly the part of the patient to be immobilised. The mask (1) is provided to exert a pre-determined force to the part of the patient to be immobilised in view of pulling this part of the patient towards the fixation plate (3, 12, 13) and of restraining the movability of the part of the patient to be immobilised within the mask.

## Description

The present invention relates to a patient immobilisation assembly for immobilising at least part of a patient, the immobilisation assembly comprising a mask made of a thermoplastic material, a fixation plate and connecting means for connecting the mask to the fixation plate in view of fixing the position of the mask with respect to the fixation plate, the mask being provided to cover at least partly the part of the patient to be immobilised, as described in the preamble of the first claim.

Devices immobilising the position of a patients' head are used to allow for an accurate and reproducible positioning of the head in medical diagnostic and treatment procedures, as for example in radiation therapy. Fractionated treatment involves the division of a radiation dose into a multiplicity of sub doses delivered to a patient on different points in time to allow for a maximum recovery of healthy tissue and to minimise complications from overexposure to radiation. In particular when exposing a patients' head to fractionated treatment, precise positioning of the target and surrounding normal structures of the head is a pre-requisite to ensure that the radiation is delivered exactly at the target position where it is needed, for example a tumor, while minimising the risk to exposure of surrounding healthy tissue.

From US-A-5.370.117 a patient immobilisation system for repeated use in imaging and treatment of brain tumors is known. The system disclosed in US-A-5.370.117 comprises an immobilisation plate onto which a positioning mask is to be fixed. On opposite sides of the immobilization plate a pair of side rails are mounted, studs protruding from the outer face of the side rails. The mask is formed over the head of the patient, holes provided along the side edges of the mask are slipped over the studs. The mask is held in place against the side rails by means of a pair of anchor bars having a multiplicity of holes adapted to engage the studs. Thus, the lower ends of the mask are received between the outer face of the side rails and the anchor bars. To prevent the mask from wobbling from side to side, the system comprises a relatively rigid arc having two vertical leg portions, the lower ends of which are secured to the outer edge of the anchor bars, the apex of the arc being connected to an upwardly disposed flap of the mask. Between the pair of side rails, a head and neck support may be slideably mounted.

This immobilisation system however has the disadvantage that it is only concerned with improving the accuracy of the positioning of the mask. However, the mask does not limit the movability of the head of the patient within the contours of the mask. This is undesirable.

From US2002/108616, a patient immobilisation system is known, which comprises a thermoplastic mask coupled to a fixation member received by the patient. The fixation member includes a mouthpiece member, part of which is to be received in the mouth of the patient. In stead of or in addition to the mouthpiece member the system may comprise at least one earplug member part of which is to be received in the patients' ear, and/or a nose block member to be positioned on the nasal bridge of the patient. Each of the afore mentioned fixation members includes fastening means securing the fixation member to the thermoplastic mask. When forming the mask, the softened thermoplastic material is placed across the face of the patient, so as to extend over the fixation members. While the thermoplastic mask cools down and hardens, a fastener plate is coupled to the fastening means so as to fix the mouthpiece member and/or earplug member and/or nose block member to the thermoplastic mask. Following cooling, the mask reflects the contours of the patients face.

This immobilisation system also presents the disadvantage that it is only considered with fixing the position of the mask with respect to the patients' face. The presence of the additional members in the mouth, ears and/or on the nose bridge are considered as uncomfortable to the patient. Also, this immobilisation device adds additional members to the mask which may in the path to be followed by the irradiation beams.

There is thus a need to an immobilisation device which goes beyond envisaging a precise positioning of the mask as such with respect to the patient. In particular there is a need to an immobilisation device which not only allows for a precise positioning of the part of the patient within the mask, but which also allows for a precise positioning with respect to a diagnostic or treatment device, during repeated and subsequent treatments or examinations, even after the patient has intermittently left the immobilisation device. When treating a patients head, a precise positioning of the target position and surrounding normal structures of the patients' head with respect to the diagnostic/treatment irradiation devices is essential, to ensure exposure of the envisaged tissue and simultaneously minimise the risk to damaging healthy tissue. This precise positioning should be obtainable even with varying dimensions of the patients head over the treatment period.

It is therefore the object of the present invention to provide an immobilisation device with which a precise positioning of the patients' part to be treated during repeated and subsequent treatments or examinations may be achieved, even with varying dimensions of the patients' part to be treated over the treatment period.

It is a particular object of this invention to provide an immobilisation device with which a precise positioning of the patients' head can be achieved, during repeated and subsequent treatments or examinations.

This is achieved according to the present invention, with the technical features of the characterising part of the first claim.

The immobilisation assembly of this invention is characterised in that the mask is provided to exert a pre-determined force to the part of the patient to be immobilised in view of pulling this part towards the fixation plate and of restraining the movability of the part of the patient to be immobilised within the mask.

An analysis of the problems occurring with existing fixation masks has revealed that a precise positioning of the fixation plate supporting the patient, with respect to the medical diagnosis or treatment device can easily be achieved. An analysis of the problems occurring with existing fixation masks has also revealed the thermoplastic mask is moulded by heating the thermoplastic material to the molten plastic state. When in the molten state, the thermoplastic material is applied to the part to be treated, for example the patients' head, shaped to follow the contours of the patients' head, fixed at three or more positions to the fixation plate and moulded by allowing the mask to cool on the patients' head for approximately 10 minutes. During this cooling, crystallisation of the thermoplastic material takes place. The crystallisation of the thermoplastic material has been found to be associated with a volume contraction of the thermoplastic material, as a result of which the mask fits well to the patients' head. However, depending on the nature of the thermoplastic material used, this fitting may become too tight when the thermoplastic material has cooled down.

Furthermore, the inventor has observed that upon storage of a thermoplastic mask for several hours or days at room temperature after having been moulded, annealing of the thermoplastic material takes place during which the thermoplastic material shows further shrinking. The inventor has also observed that upon annealing the degree of shrinking depends on the nature of the material, as well as on the geometrical size and design of the polymer sheet. When applied to the patients' head after a few days, as a result of the additional shrinking occurred during annealing, often the mask fits too tightly to the head, thus rendering it uncomfortable. This is unwanted.

The inventor has further observed that in the course of time the dimensions of the patients' head may diminish when the patient is loosing weight, or increase when the patient is gaining weight, or shows swelling for other reasons. This has the consequence that either the patient will be able to move his head within the mask, which adversely affects the accuracy of the positioning of the head, or that the fitting of the mask will become more and more tight and less comfortable.

Once insight in these problems had been obtained, it became clear that the problem of a too tight fitting as well as the problem of a too loose fitting could be solved by making use of a mask which is provided to exert a pulling force of a pre-determined magnitude to the part of the patient to be treated. The pulling force is arranged to pull the part of the patient to be treated towards the fixation plate, in view of restraining the movability of the part within the mask.

Because a pre-determined pulling force is exerted, which is kept as constant as possible during repeated and subsequent treatments, it can be ensured that a precise positioning of the part to be treated can be achieved during such repeated and subsequent treatments or examinations. This precise positioning can be obtained even if the patient has left the immobilisation device and returns to it, after a few hours, days or even weeks have lapsed. This precise positioning allows for a selective treatment and examination of the target position, while leaving surrounding normal structures of the patient unaffected. Especially when treating the head, a precise positioning is essential, as a slightly wrong positioning may involve the loss of essential functions within the brain tissue.

In general, it is preferred that the fixation force exerted by the mask is about 75-200 N, preferably 100-150 N, as within this range a good compromise is provided between the reproducibility of the patient positioning and the patient comfort. Depending on the part of the body to be treated, this force may be increased or reduced.

A preferred embodiment of the immobilisation assembly of this invention is characterised in that it comprises means for adjusting the pulling force exerted by the mask to the immobilised part of the patient. In case of too loose fitting, fitting of the mask may be improved by shortening the distance between the mask and the patients' part covered by the mask. Shortening of the distance will in general increase the pulling force. In case of too tight fitting, the mask may be loosened to allow for example for a movement of maximum approximately 1 mm, to improve comfort to the patient without this going at the expense of the positioning precision. This may be particularly relevant when irradiating the head.

An optimum application of the pulling force has been found to occur if the pulling force is exerted to the edges, along which the mask is connected to the fixation plate. The pulling force may be exerted to the edges, either directly or indirectly through the connecting means connecting the mask to the fixation plate.

Another preferred embodiment of the immobilisation assembly of this invention is characterised in that the means for adjusting the pulling force exerted by the mask comprise means for varying the distance of the mask with respect to the immobilised part of the patient. This may be achieved by any suitable means known to the man skilled in the art and allows to control the magnitude of the force exerted by the mask, to adjust this force in subsequent treatments and keep it virtually constant in view of providing high stability and reproducibility of the patient positioning and comfort.

Still another preferred embodiment of this invention is characterised in that the means for adjusting the pulling force exerted by the mask comprise means for displacing the patients' head with respect to the mask.

The invention and additional preferred embodiments are further elucidated in the appending figures and description of the figures.
Figure 1 is a view to a preferred embodiment of the assembly of this invention.
Figure 2 is a cross section of the assembly of this invention, showing the fixation of the mask to the support plate in the presence of a spacer.
Figure 3, 4 and 5 show a view to a fixation plate comprising a top and bottom face which are spaced from each other using different types of spacers.
Figure 6 is a detailed view to a fixation plate comprising a top and bottom face which are spaced from each other using adjustable spacers.

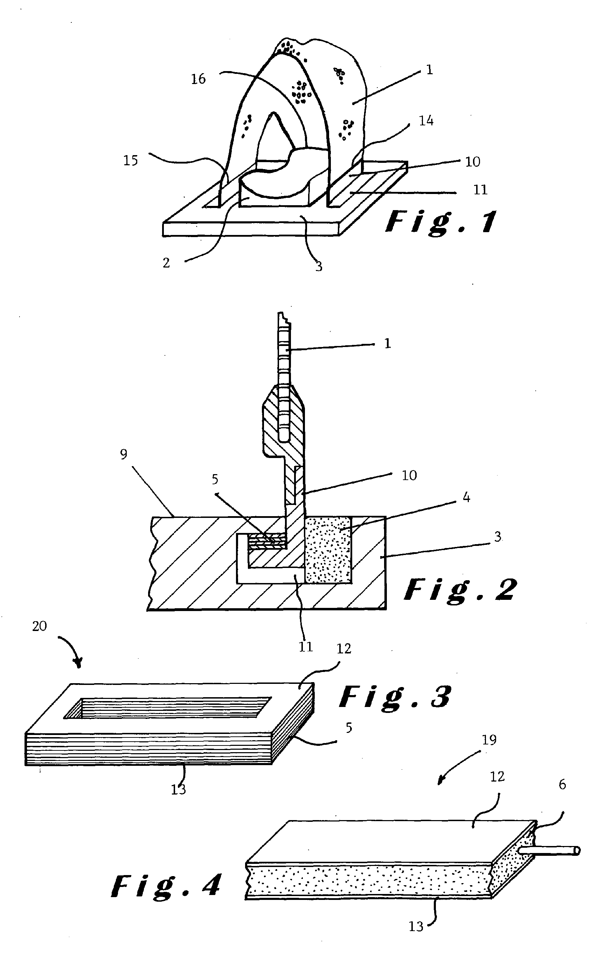

The embodiment of the assembly of this invention shown in figure 1 comprises a mask 1, the position of which is fixed by connecting it to a fixation plate 3. In the embodiment shown, the mask 1 is provided to cover at least part of the head of a patient.

The mask 1 shown in figure 1 is preferably made of a thermoplastic material, which is moulded by positioning the patients' head onto the fixation plate. If so desired, to improve comfort to the patient and to improve the patient positioning and reproducibility thereof, the head and neck may be supported by a head and neck support 2.

To allow positioning the head or any other part of the patient to be treated/examined by irradiation as accurately as possible, even with fractionated treatment, the mask 1 is moulded to the part of the patient to be treated. For the sake of simplicity and because of the particular importance of an accurate positioning when treating the head of a patient, hereafter reference will be made to a mask for fixing the position of the head of a patient.

When moulding the mask, the thermoplastic material is heated to the molten plastic state, where the thermoplastic material becomes sufficiently flexible to be shaped over the patients' head. When molten, the thermoplastic material is applied to the patients' head and shaped to follow the contours of the head. The opposite longitudinal edges 14, 15 of the mask 1 which extend along the patients' face, as well as the transversal edge 16 which extends along the top of the patients' head are fixed to the fixation plate 3.

To fix the mask 1 to the fixation plate 3, as is shown in figure 2, to the opposite longitudinal 14, 15 and transversal 16 edges of the mask each time a rail 10 is connected, which is provided to co-operate with a corresponding groove 11 provided in the fixation plate 3. However, any other suitable connecting means known to the person skilled in the art for connecting the mask 1 to the fixation plate 3, may be used. Also, if so desired the mask 1 may be fixed at other or additional positions. Usually the nature and position of the connecting means will be adapted depending on the nature of the body part to be immobilised by the mask 1.

The mask 1 is allowed to cool on the patients' head to allow for a moulding and a crystallisation of the thermoplastic material, for approximately 10 minutes. The crystallisation of the thermoplastic material has been found to be associated with a volume contraction of the material as a result of which the mask shrinks and fits well to the patients' head. As a consequence of this shrinking the length of the material extending between the opposite longitudinal edges 14, 15 of the mask 1 is reduced and a force is exerted to the part of the patient covered by the mask 1. Depending on the nature of the thermoplastic material used, this fitting may be either insufficient and allow for a displacement of the patients' head within the mask, or become too tight. Also, when stored for some more time at room temperature, annealing of the material takes place which has been found to be associated with a further shrinking of the mask. The additional shrinking has the consequence that the mask fits too tightly to the patients' head, which is uncomfortable, and that the positioning of the patient with respect to the radiation instrument may have changed somewhat, which is undesirable.

Therefore, with the present invention means are provided with which the mask 1 may exert a pre-determined force to the part of the patient covered by the mask 1, in view of pulling the immobilised part of the patient towards the fixation plate 3 and of restraining the movability of the part of the patient immobilised within the mask 1. The pulling force is preferably adjustable so as to allow increasing or reducing it depending on the specific circumstances. Thereto means are provided which allow adapting the pulling force exerted by the mask either in a continuous manner or in discrete steps.

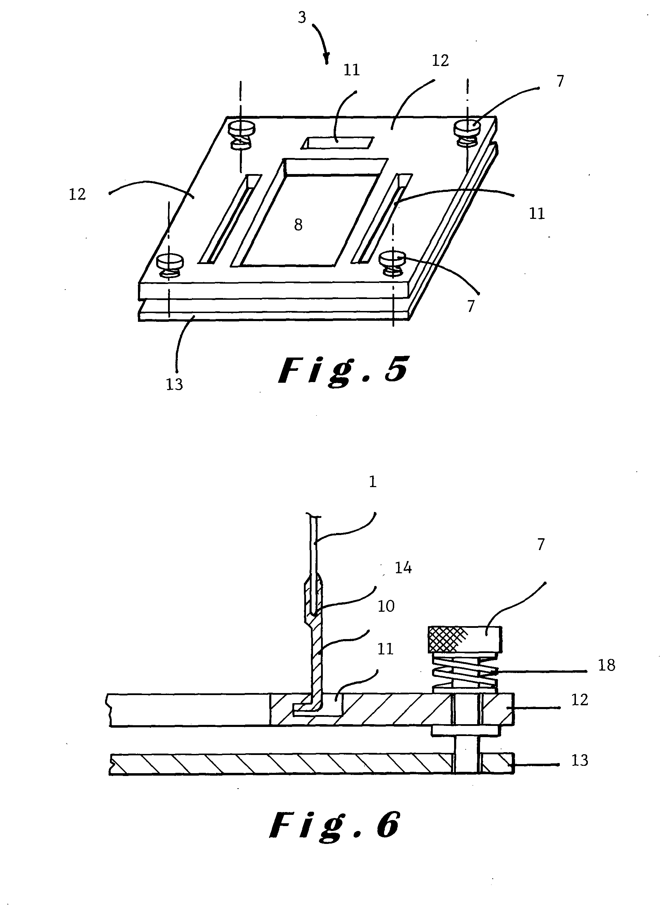

To allow adjusting the pulling force exerted by the mask 1, the assembly of this invention comprises means 2, 5, 6, 7, 10, 11, 12, 13, 14, 15, 16 for adjusting the position or distance of the mask 1, the fixation plate 3 and/or the head and neck support 2 with respect to each other. It is preferred that the distance varying means are adjustable in such a way that a virtually constant pulling or fixation force may be applied by the mask 1 to the patients' head, to ensure optimum reproducibility of the patient positioning not only within the mask but also with respect to the fixation plate 3 and medical diagnostic or treatment device, and at the same time maximising patient comfort. The distance varying means may either be continuously adjustable as for example shown in figure 4, 5, 6, or may be adjustable in discrete steps by means of a spacer 5, 20 as is shown in figure 2, 3.

The pulling force exerted by the mask 1 is preferably varied by varying the distance between the mask and the patients' head which is supported by the fixation plate 3 and/or head and neck support 2. This distance may be varied by providing means with which either
1. the distance of the mask 1 with respect to the fixation plate 3, 12, 13 may be varied, i.e. positioning the mask 1 in a moveable manner with respect to the fixation plate 3, 12, 13;
2. the position of the fixation plate 3, 12, 13 with respect to the mask 1 may be varied. If use is made of a head and neck support 2, this will usually have the consequence that also the position of the head and neck support 2 with respect to the mask 1 is varied.
3. the position of the head and neck support 2 with respect to the mask 1 may be varied, i.e. positioning the head and neck support 2 in a moveable manner or moveably positioning either the fixation plate 3 or the mask 1 with respect to the head and neck support 2
4. the position of the mask 1 and support plate 3 with respect to the head and neck support 2 may be varied.
However, also any combination of the above described features may be used, depending on the envisaged use of the assembly.

As is shown in figure 2, the pulling force exerted by the mask 1 and the position of the mask with respect to the fixation plate 3 may for example be varied by varying the position in height direction of the device, in which the rail 10 is inserted in the groove. When inserted lower in the groove 11, further away from the support surface 9 supporting the head, the pulling force will be increased. When inserted closer to the support surface 9, the pulling force will be decreased. The positioning of the rail 10 within the groove 11 may for example be varied by using spacers 5, taking the form of inserts which are to be inserted in the groove 11 or removed therefrom. Any space remaining in the groove 11 may be filled with an additional member 4.

In the embodiment shown in figure 3, either the head as such or the head and neck support 2 is provided to be positioned on top of a spacer 20, positioned on top of the fixation plate 3. The spacer 20 comprises a plurality of super imposed plates 5. The thickness of the spacer, and thus the distance between the fixation plate 2 and the mask may be varied by adding or subtracting one or more plates 5. By increasing the number of plates 5, the force applied by the mask 1 to the patients head may be increased. By decreasing the number of plates, the force applied by the mask to the patients head may be decreased. Usually lifting or lowering of the patients head by approximately 1-5 mm, preferably 1-3 mm will be sufficient to release a too tight force or to increase the force applied by the mask. However, depending on the part of the patient to be treated, this distance may be smaller or larger. As can be seen from figure 3, the spacer 20 may be hollow in the centre, so that the patient may be irradiated from all directions with minimum loss of radiation intensity.

In the embodiment shown in figure 4, the fixation plate comprises an upper and a lower surface 12, 13 which are spaced from each other by means of a spacer, which in this case is formed by an expandable member 6. The height of the expandable member 6 is variable. By expanding or contracting the member, the patient will be moved towards and from the mask, thus increasing or reducing the force exerted by the mask to the patients' head. Expansion or contraction of the member 6 may be achieved in any suitable manner known to the man skilled in the art, for example in a pneumatic or a hydraulic manner. However, the device 19 shown in figure 4 may also be used as a spacer which is provided for positioning between the fixation plate 3 and the head and neck support 2.

In the embodiment shown in figure 5, the top and bottom surface of the fixation plate 3 are displaceable to and from each other by means of a spacers 7 having a continuously variable height. In the embodiment shown in figure 5, the top surface 12 of the fixation plate is provided for receiving the connecting means for connecting the mask to the fixation plate, whereas the head and neck support is provided to be positioned in the cavity 8 provided in the top surface 12, and to be supported by the bottom surface 13.

In the embodiment shown in figure 6, opposite longitudinal edges 15 of the mask 1 are each connected to connecting means 10. The connecting means 10 are provided to be releasably fixed in a groove 11 provided in the fixation plate 3. The fixation plate 3 comprises a top plate 12 and a bottom plate 13, the top plate 12 being displaceable towards and from the bottom plate 13, in view of increasing or decreasing the pulling force exerted by the mask 1. In the case shown in figure 6, the distance between the top and bottom plate is continuously variable by means of screws 17, the position of the top plate being fixed with respect to the screw 17 by means of a resilient member 18. The spring constant of the resilient member 18 will usually be selected so as to obtain the desired fixation force.

If so desired the distance between the fixation plate 3 and the mask 1 may also be varied by positioning on top of the fixation plate 3 an expandable member, which is provided to support the patients' head and/or the head and neck support 2. Or else, the head and neck support may be made of an expandable material.

The mask 1 will generally be made of a thermoplastic material, for example poly-ε-caprolacton, polyurethane, or other types of polyester. However, a material having a different shrinking coefficient upon crystallisation may also be used. The shrinking force exerted by the mask during cooling may be measured by means of the device disclosed in Belgian patent application n°2002/05012808.

The patient immobilisation assembly of this invention presents the advantage that it does not add any parts to the existing systems, which otherwise could interfere with the radiation directed to the patient. It is of utmost importance that radiation originating from various directions can be directed to the patient, to allow for an optimum treatment. With the presence of interfering parts, it may be required to adapt the instrumentation and planning of the treatment. This is unwanted.

The invention also relates to the mask, the above described fixation plate, expandable fixation plate, expandable mask, expandable spacer and expandable head and neck support for use with the above described assembly.

## Claims

1. A patient immobilisation assembly for immobilising at least part of a patient, the immobilisation assembly comprising a mask (1) made of a thermoplastic material, a fixation plate (3, 12, 13) and connecting means (10, 11, 14, 15, 16) for connecting the mask (1) to the fixation plate (3, 12, 13) in view of fixing the position of the mask with respect to the fixation plate, the mask being provided to cover at least partly the part of the patient to be immobilised, **characterised in that** the mask (1) is provided to exert a pre-determined force to the part of the patient to be immobilised in view of pulling this part of the patient towards the fixation plate (3, 12, 13) and of restraining the movability of the part of the patient to be immobilised within the mask.

2. An assembly as claimed in claim 1, **characterised in that** the immobilisation device comprises means (5, 6, 7, 17, 18) for adjusting the pulling force exerted by the mask (1) to the immobilised part of the patient.

3. An assembly as claimed in claim 1 or 2, **characterised in that** the mask comprises at least one edge (14), **in that** the connecting means (10) for connecting the mask (1) to the fixation plate (3, 12, 13) are connected to the at least one edge (14, 15, 16) of the mask (1) and **in that** the pulling force is exerted to the at least one edge of the mask.

4. An assembly as claimed in any one of claims 1-3, **characterised in that** the means (5, 6, 7, 17, 18) for adjusting the pulling force exerted by the mask (1) comprise means for varying the distance between the part of the mask covering the patient and the part of the patient to be immobilised.

5. An assembly as claimed in any one of claims 1-4, **characterised in that** the means (5, 6, 7, 17, 18) for adjusting the pulling force exerted by the mask (1) comprise means for displacing the mask with respect to the patient, in view of varying the distance of the part of the mask covering the patient with respect to the part of the patient to be immobilised.

6. An assembly as claimed in any one of claims 1-5, **characterised in that** the fixation plate (3, 12, 13) comprises a top surface (12) and bottom surface (13), **in that** the mask is connected to the top surface (12) and **in that** means (6, 7) are provided for moving the top surface with respect to the bottom surface.

7. An assembly as claimed in any one of claims 1-5, **characterised in that** the fixation plate (3, 12, 13) comprises a top surface (12) and bottom surface (13), **in that** the mask is connected to the bottom surface (13) and **in that** means (6, 7) are provided for moving the bottom surface (13) with respect to the top surface (12).

8. An assembly as claimed in any one of claims 1-7, **characterised in that** the assembly comprises a support (2), the support being provided for positioning on top of the fixation plate (3, 12, 13) in view of supporting the part of the patient to be immobilised, and **in that** the means for adjusting the force comprise means for varying the position of the support (2) with respect to the mask (1).

9. An assembly as claimed in any one of claims 1- 8, **characterised in that** the assembly comprises a support (2) for supporting the part of the patient to be treated, the support (2) being positioned independently of the fixation plate (3), and **in that** the means for adjusting the force comprise means for varying the position of the support (2) with respect to the mask (1) and/or the fixation plate (3).

10. An assembly as claimed in any one of claims 1-9, **characterised in that** the mask (1) is provided to immobilise the head of the patient.

11. An assembly as claimed in any one of claims 1-10, **characterised in that** the device comprises a head and neck support (2) for supporting the head and neck of a patient.
